# EUROPEAN PATENT SPECIFICATION

(11) **EP 1 974 746 B1**
(45) Date of publication and mention of the grant of the patent: **11.01.2012**
(21) Application number: 08153545.2
(22) Date of filing: 28.03.2008
(51) Int. Cl.: A61K 39/00

(54) **Use of an adjuvanted allergy vaccine formulation for parenteral administration**
Verwendung einer hilfsstoffhaltigen Impfstoffformulierung für parenterale Verabreichung
Utilisation de formule adjuvante de vaccin contre les allergies pour administration parentérale

(30) Priority: 28.03.2007 US 907317 P; 28.03.2007 EP 07105094
(43) Date of publication of application: 01.10.2008
(73) Proprietor: Alk-Abelló A/S, 2970 Hørsholm (DK)
(72) Inventor: Wüstenberg, Eike Gunther, 22589 Hamburg (DE)
(74) Representative: Inspicos A/S

(56) References cited:
- WO-A-2005/077410
- WO-A-2005/083385
- SEIDENBERG J ET AL: "EVALUATION OF THE SAFETY OF A SEASONAL ULTRA RUSH HIGH-DOSE SUBLINGUAL IMMUNOTHERAPY IN CHILDREN WITH ALLERGIC RHINITIS TO TREE POLLEN AND GRASS POLLEN" CLINICAL AND EXPERIMENTAL ALLERGY, BLACKWELL SCIENTIFIC PUBLICATIONS, LONDON, GB, vol. 36, no. 9, September 2006 (2006-09), pages 1205-1206, XP009085031 ISSN: 0954-7894
- ON BEHALF OF THE UK IMMUNOTHERAPY STUDY GROUP FREW ET AL: "Efficacy and safety of specific immunotherapy with SQ allergen extract in treatment-resistant seasonal allergic rhinoconjunctivitis" JOURNAL OF ALLERGY AND CLINICAL IMMUNOLOGY, MOSBY - YEARLY BOOK, INC, US, vol. 117, no. 2, February 2006 (2006-02), pages 319-325, XP005275416 ISSN: 0091-6749
- NIGGEMANN B ET AL: "Five-year follow-up on the PAT study: specific immunotherapy and long-term prevention of asthma in children" ALLERGY (OXFORD), vol. 61, no. 7, July 2006 (2006-07), pages 855-859, XP002437350 ISSN: 0105-4538
- LEE L.K. ET AL: "AGGRESSIVE COSEASONAL IMMUNOTHERAPY IN MOUNTAIN CEDAR POLLEN ALLERGY", ARCHIVES OF OTOLARYNGOLOGY, vol. 108, no. 12, 1982, pages 787-794, XP008115849, CHICAGO, USA

## Description

### TECHNICAL FIELD

The present invention relates to a seasonal allergen composition for the manufacture of a vaccine formulation for preventing or treating allergy in a subject by parenteral administration, wherein the vaccine formulation is administered in a dosage regimen comprising an up-dosing phase and a maintenance phase.

### BACKGROUND OF THE INVENTION

Allergy is a major health problem in countries where Western lifestyle is adapted. Furthermore, the prevalence of allergic disease is increasing in these countries. Although allergy in general may not be considered a life-threatening disease it can progress into asthma, which annually causes a significant number of deaths. An exceptional prevalence of about 30% in teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. Firstly, a progression of symptoms and disease severity, as well as disease progression, for example from hay fever to asthma. Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen sources are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis yet other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. It therefore follows that a foreign substance having only one antibody binding epitope does not initiate an allergic reaction. The cross-linking of receptor bound IgE on the surface of mast-cells also leads to release of signaling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with by the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, neither do they control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Specific allergy vaccination is, in spite of its virtues, not in widespread use, primarily for two reasons. One reason is the inconveniences associated with the traditional vaccination programme that comprises repeated vaccinations i.a. injections over a several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment.

For seasonal allergens, such as allergens from tree pollen, grass pollen, weed pollen and fungi, up-dosing is usually started and completed well in advance of the season to avoid initiating treatment of patients with on-going symptoms and hence to minimise the risk of side effects.

Attempts to improve vaccines for specific allergy vaccination have been performed for over 30 years and include multifarious approaches. Several approaches have addressed the allergen itself through modification of the IgE reactivity. Others have addressed the route of administration and the protocol regimen used.

Seidenberg et al. (Clinical and Experimental Allergy, 36, 1201-1212, British Society for Allergy and Clinical Immunology, Annual Conference - July 2006, Abstract S17) describes a study of immunotherapy treatment of children with a liquid allergy vaccine administered sublingually, wherein the treatment is initiated shortly before the start of the season or during the season. Lee et al (Archives of Otolaryngology, vol. 108, no. 12, 1982, pages 787-794) discloses a treatment with injections of the mountain cedar extract commencing after the start of the allergen season.

The object of the present invention is to provide an improved method for preventing or treating allergy in a subject using an adjuvanted vaccine formulation for parenteral administration.

### SUMMARY OF THE INVENTION

This object is obtained by the present invention, which relates to the use of a grass pollen allergen composition for the manufacturing of a vaccine formulation for preventing or treating allergy to grass pollen allergen in a subject by subcutaneous administration, wherein the vaccine formulation comprises an adjuvant selected from the group of oxygen-containing metal salts and is administered in a dosage regimen comprising an up-dosing phase, wherein the up-dosing phase is initiated after start of the allergen season of the allergen composition and wherein allergen season means a period of time, the start of which is the first of three consecutive days, wherein the level of airborne allergen-.. containing particles is above a threshold value, which is 5 % of the average peak value of the previous 10 years, at one or more measuring locations in the region or country, and the end of which is on the last or three consecutive days, wherein the lever of airborne allergen-containing particles is below a threshold value, which is 5 or the average peak value of the previous 10 years at one or more measuring locations in the region or country

Alternatively worded, the present invention relates to a vaccine formulation comprising a grass pollen allergen- for use in preventing or treating allergy to grass pollen allergen fin a subject by subcutaneous administration, wherein the vaccine formulation comprises an adjuvant selected from the group of oxygen-containing metal salts and is administered in a dosage regimen comprising an up-dousing phase, wherein the up-dasing phase is initiated after start of the allergen season of the allergen- composition and wherein allergen- season means a period of time, the start of which is the first of three consecutive days, wherein the level of airborne allergen-containing particles is above a threshold value, which is 5 % af the average peak vale of the previous 10 years, at one or more measuring locations in the region or country, and the end of which is on the last of three consecutive days, wherein the level of airborne allergen-containing particles is below a threshold value, which is 5 % of the average peak value of the previous 1.0 years at one or more measuring locations in the region or country.

The present invention is based on clinical results, which surprisingly have shown that allergy vaccination for the treatment of allergy to seasonal allergens by subcutaneous administration may be initiated within the allergen season while maintaining an acceptable level of side effects. This finding is very surprising in the sense that it has hitherto been believed that for seasonal allergens it is necessary to carry out the full up-dosing phase prior to the season to avoid serious side effects.

It has been found that a high proportion of patients in need of allergy vaccination do not go to their doctor until they feel the symptoms of the allergy, i.e. in the season for seasonal allergens. Until now, it has not been possible to initiate parenteral allergy vaccination within the season, and therefore the treatment of patients with on-going symptoms in the season has to be postponed to a time after the season with the result that the patients have to endure symptoms for a longer period and risk that the allergy deteriorates. Moreover, many patients are not motivated to start treatment outside the season, where they do not experience any inconvenience of the allergy, and hence they will not initiate treatment in due time before the next allergen season.

The possibility of initiating subcutaneous allergy vaccination within or up to the allergen season makes the dosage regimen much simpler for both patients and doctors and allergists treating the patients and will facilitate that a lot more people will receive allergy vaccination.

### DETAILED DESCRIPTION OF THE INVENTION

### Allergen composition

In a preferred embodiment of the invention, the allergen composition incorporated into the formulation is selected from the group consisting of an allergen extract, a purified fraction of an allergen extract, a modified allergen, a recombinant allergen and a mutant of a recombinant allergen. An allergenic extract may naturally contain one or more isoforms of the same allergen, whereas a recombinant allergen typically only represents one isoform of an allergen. The mutant allergen may be a low IgE-binding mutant, e.g. a low IgE-binding allergen according to WO 99/47680, WO 02/40676 or WO 03/096869 A2. The modified allergen may be any allergen derivative modified by e.g. chemical, physical or enzymatic treatment, including e.g. allergoids. In a preferred embodiment the allergen is in the form of an extract. In another preferred embodiment the allergen is a recombinant allergen. In a further preferred embodiment the allergen is an allergoid. In a further preferred embodiment the allergen is a naturally occurring low IgE-binding mutant or a recombinant low IgE-binding mutant. Furthermore, the allergen may be a mixture of a number of allergens, e.g. from 2 to 10 allergens, in particular from 3 to 9 allergens, more in particular from 4 to 8, and most in particular from 5 to 7 allergens.

Important pollen allergens from grasses are such originating from the taxonomic orders of Poales including i.a. grasses of the genera Lolium, Phleum, Poa, Cynodon, Dactylis, Holcus, Phalaris, Secale, and Sorghum.

In a particular embodiment of the invention the allergen is Phl p 1, Phl p 5, Phl p 6, Poa p 1, Poa p 5, Sec c 1, Sec c 5 or Sor h 1.

In yet another embodiment of the invention the composition comprises at least two different types of allergens originating from the same allergic source, such as grass group 1 and grass group 5 allergens from different grass species.

When the allergen composition comprises more than one allergen, the allergens may be present in equi-molar amounts or the ratio of the allergens present may vary preferably up to 1:20.

The preferred maintenance phase potency of a mono-dose formulation is from 100 to 1000000 SQ-u, more preferably from 500 to 500000 SQ-u, more preferably from 1000 to 300000 SQ-u, more preferably from 10000 to 200000 SQ-u, more preferably from 50000 to 150000 SQ-u and most preferable from 75000 to 125000 SQ-u.

The amount of allergen, which corresponds to a given level of potency, varies strongly depending on the allergen specie. In a further embodiment of the invention the concentration of major allergen in a mono-dose is from 0.05 to 50 µg, more preferably from 0.05 µg to 30 µg, more preferably from 0.06 µg to 25 µg, more preferably from 0.07 µg to 20 µg, more preferably from 0.08 µg to 15 µg, more preferably from 0.09 µg to 10 µg and most preferably from 0.1 ug to 7 µg.

### Administration

The period of administration during the up-dosing phase and the maintenance phase may be a continuous period. Alternatively, the period of administration is a discontinuous period interrupted by one or more periods of non-administration. Preferably, the (total) period of non-administration is shorter than the (total) period of administration.

### Up-dosing phase and maintenance phase

The dosage regimen used in the present invention may be any conventional dosage regimen in respect to dosing and number, period and frequency of administration.

In one aspect of the invention, the dosage regimen comprises a maintenance phase following the up-dosing phase.

In a particular embodiment of the invention, the duration of the up-dosing phase is from 1 week to 30 weeks, preferably from 6 weeks to 25 weeks, more preferably from 10 weeks to 20 weeks, and most preferably from 13 week to 17 weeks.

In another particular embodiment of the invention, the number of administrations in the up-dosing phase is from 1 to 30, preferably from 5 to 25, more preferably from 10 to 20, and most preferably from 13 to 18.

In another particular embodiment of the invention, the administrations in the up-dosing phase are carried out with intervals of from 1 to 14 days, preferably from 1 to 11 days, more preferably from 1 to 9 days, more preferably from 1 to 8 days, and most preferably from 2 to 8 days.

Preferably, the up-dosing phase includes a number of dose levels of from 1 to 8, preferably from 2 to 7, more preferably from 3 to 6, and most preferably from 4 to 5.

In another particular embodiment of the invention, before the end of the allergen season, the up-dosing has been progressed to a level, wherein the dose administered is at least 1 %, preferably at least 2 %, preferably at least 5 %, preferably at least 10 %, preferably at least 20 %, preferably at least 30 %, preferably at least 40 %, preferably at least 50 %, preferably at least 60 %, preferably at least 70 %, preferably at least 80 % and most preferably at least 90 % of the maintenance dose. In a preferred embodiment of the invention, before the end of the allergen season the up-dosing has been progressed to the level of the maintenance dose, i.e. the up-dosing phase has been completed within the allergen season.

In another particular embodiment of the invention, the duration of the maintenance phase is from 12 months to 48 months, preferably from 24 months to 42 months, more preferably from 30 months to 40 months, and most preferably from 34 months to 38 months.

In another particular embodiment of the invention, the number of administrations in the maintenance phase is from 9 to 34, preferably from 18 to 32, more preferably from 22 to 30, most preferably from 24 to 28.

In another particular embodiment of the invention, the administrations in the maintenance phase are carried out with intervals of from 1 to 14 weeks, preferably from 3 to 10 weeks, more preferably from 4 to 8 weeks, most preferably from 5 to 7 weeks.

In one particular embodiment of the invention, the up-dosing phase is initiated within the allergen season. The up-dosing phase may be initiated at the start of the allergen season or after the start of the season. In particular, the up-dosing phase is initiated more than 1 week, more particularly more than 2 weeks, more particularly more than 3 weeks, more particularly more than 4 weeks, more particularly more than 5 weeks, more particularly more than 6 weeks, more particularly more than 10 weeks after the start of the allergen season.

In a second aspect of the invention the dosage regimen does not comprise a maintenance phase following the up-dosing phase. In this aspect the dosage regimen preferably comprises from 1 to 10 up-dosing phases, more preferably from 2-8 up-dosing phase and most preferably from 3 to 6 up-dosing phases.

In a particular embodiment of the second aspect of the invention, the duration of each up-dosing phase is from 1 week to 15 weeks, preferably from 3 weeks to 12 weeks, more preferably from 4 weeks to 10 weeks, and most preferably from 5 weeks to 9 weeks. In another particular embodiment of the invention, the number of administrations in each up-dosing phase is from 1 to 15, preferably from 3 to 12, more preferably from 4 to 10, and most preferably from 5 to 9. In another particular embodiment of the invention, the administrations in the up-dosing phase are carried out with intervals of from 1 to 14 days, preferably from 2 to 12 days, more preferably from 3 to 11 days, more preferably from 4 to 10 days, and most preferably from 5 to 9 days. Preferably, the up-dosing includes a number of dose levels of from 1 to 14, preferably from 3 to 12, more preferably from 4 to 10, and most preferably from 5 to 9.

### Vaccine formulation

The vaccine formulation according to the present invention may be any formulation suitable for parenteral administration, including a mixture, a solution, a suspension, a dispersion, an emulsion, a gel, a paste, a syrup, a cream, an ointment, implants, depositories and a re-dissolvable powder, granulate or lyophilisate, preferably a solution or a re-dissolvable lyophilisate.

In a particular embodiment of the vaccine formulation of the invention, the vaccine formulation has been prepared by lyophilising a solution of the allergen composition, wherein the vaccine formulation is adapted for being re-dissolved prior to administration.

The vaccine formulation of the invention may further comprise any adjuvant and other excipients suitable for such type of formulation. Such excipients are well-known to the person skilled in the art and include i.a. solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1β, IL-2, IL-7, IL-12, INFγ), GM-CSF, MDF derivatives, CpG oligonucleotides, LPS, MPL, phosphophazenes, Adju-Phos®, glucan, antigen formulation, liposomes, DDE, DHEA, DMPC, DMPG, DOC/Alum Complex, Freund's incomplete adjuvant, ISCOMs®, LT Oral Adjuvant, muramyl dipeptide, monophosphoryl lipid A, muramyl tripeptide, and phospatidylethanolamine.

Aqueous solutions of oxygen-containing metal salts typically have the form of gels. The concentration of aluminium hydroxide in the formulation is preferably 0.035-1000 mg/ml, more preferably 0.10-100 mg/ml, more preferably 0.25-10 mg/ml, and most preferably 0.5-5 mg/ml. However, the formulation of the invention may also have the form of a highly concentrated gel or gel-like formulation.

The formulation of the present invention may be prepared by dissolving the adjuvants and other excipients, if any, in a solvent, preferably water, adding the allergen and allowing the allergen and adjuvants and other excipients to react for a period of time. The reaction period may be from 0.1 to 48 hours, preferably from 12 to 24 hours. The reaction is preferably carried out at a temperature of from 4 to 45 °C, more preferably from 4 to 20 °C.

### Oxygen-containing metal salts

In a preferred embodiment of the invention, the vaccine formulation of the invention comprises an oxygen-containing metal salt adjuvant. Preferably, the metal cation of the oxygen-containing metal salt is selected from the group consisting of Al, K, Ca, Mg, Zn, Ba, Na, Li, B, Be, Fe, Si, Co, Cu, Ni, Ag, Au and Cr.

The anion of the oxygen-containing compound may be any oxygen-containing anion, including an organic or inorganic anion, or a combination of organic and inorganic anions. Examples of suitable oxygen-containing metal salts are e.g. those, wherein the anion is selected from the group consisting of sulphates, hydroxides, phosphates, nitrates, iodates, bromates, carbonates, hydrates, acetates, citrates, oxalates, and tartrates, as well as mixed forms thereof. The oxygen-containing metal salts further comprise coordination complexes. A definition of coordination complexes is given in e.g. The Handbook of Chemistry and Physics 56 Ed., Section B, Chapter 7 (197576).

Within the present context, the expression "mixed forms" is intended to include combinations of the various anions as well as combinations with e. g. chlorides, and sulphides.

Examples of oxygen-containing metal salts according to the invention are aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate, and barium sulphate.

Most preferred are aluminium hydroxide, aluminium phosphate, aluminium acetate, calcium phosphate, calcium tartrate and zinc sulphate.

The pl of the oxygen-containing metal salt is typically in the range of 2-11. The pl for allergen proteins is typically in the range of 4-9. Preferably, the allergen and oxygen-containing metal salt are selected so that the pl of the allergen is lower than the pl of the oxygen-containing metal salt.

When using e.g. aluminium hydroxide as oxygen-containing metal salt, the concentration of aluminium hydroxide in the formulation is preferably 0.035-1000 mg/ml, more preferably 0.10-100 mg/ml, more preferably 0.25-10 mg/ml, and most preferably 0.5-5 mg/ml. For the other oxygen-containing metal salts, the concentration of the metal salt is preferably 0.035-1000 mg/ml, more preferably 0.35-100 mg/ml, more preferably 0.7-50 mg/ml, and most preferably 1.0-20 mg/ml. The concentration of allergen in the formulation is preferably 0.01-100 mg/ml, more preferably 0.1-10 mg/ml. The ratio of oxygen-containing metal salt to allergen is preferably from 0.1 to 100, more preferably from 1 to 20. The degree of allergen adsorbed to the oxygen-containing metal salt is typically from 5 to 99 %, more preferably from 10 to 99 % of the added amount. The adsorption of allergen to the oxygen-containing metal salt depends on the buffer system and the reaction conditions, including temperature and reaction time, under which the adsorption takes place.

Oxygen-containing metal salts can be characterised by a variety of physical-chemical parameters like adsorption, solubility and dissolution properties, ionic charge measured as the isoelectric point pl (pH where the net charge of the substance is zero for a dissociationable compound), dissociation constants, complex coordination, electronic configurations, valence, bonding orbitals and antibonding orbitals, depot properties, adhesion properties, surface characteristics, particle characteristics, and adjuvanticity.

It is believed that the biologically active substance is adsorbed (or coupled) to the oxygen-containing metal salt, and this adsorption contributes to the efficacy of the vaccine. Several factors may be important or influence the adsorption between the active substance and the oxygen-containing metal salt (see e. g. P. M. Callahan et al., Pharmaceutical Research Vol. 8, No. 7,851-858 (1991), and Vaccine Design. The Subunit and Adjuvant Approach). These factors include pH, the length of time the adsorption reaction is carried out for, mixing conditions, concentrations of the various components in the vaccines, containers, temperature, storage, buffer and excipients. It has further been found that the adsorption of the active substance may be influenced by the net/overall charge of the metal salt and the charge of the active substance, both of which are pH dependent. A further feature believed to be of importance is the solubility of the oxygen-containing metal salts.

Another feature of oxygen-containing salts is the protection of the active substance either by maintaining the ideal pH for the active substance in the microenvironment, thus preventing acid degradation, or by protecting the active substance against enzymatic degradation thereby allowing the substance to be delivered.

Furthermore, some of the oxygen-containing metal salts have a buffer capacity. This may result in an in vivo microenvironment within the vaccine formulation, which protects the active substance from the degradable environment.

A further feature of the oxygen-containing metal salt (s) is their capability to adhere to the mucosal membrane. This is believed to increase absorption of the allergen through the mucosal membrane.

For several of the oxygen-containing metal salts (e. g. AI(OH)₃, AIPO₄, Ca₃PO₄) the particle size range is between 0.5 and15 µm.

### DEFINITIONS

In connection with the present invention the following definitions are used:

The term "allergen composition" means any composition containing one or more allergens, including biological and biologically derived compositions and compositions containing synthetic allergens.

The term "up-dosing phase" means a period of treatment during which the doses of allergen composition administered are gradually increased to reach a full dose level, which is used in the following maintenance phase, and the up-dosing phase ends when the said full dose level is reached, i.e. immediately subsequent to the administration of the first full dose.

The term "maintenance phase" means a period of treatment in continuation of the up-dosing phase and during which a full dose of allergen composition is administered, the maintenance phase starting immediately subsequent to the administration of the first full dose.

The term "allergen season" means a period of time, the start of which is the first of three consecutive days, wherein the level of airborne allergen-containing particles is above a threshold value, which is 5 % of the average peak value of the previous 10 years, at one or more measuring locations in the region or country, and the end of which is on the last of three consecutive days, wherein the level of airborne allergen-containing particles is below a threshold value, which is 5 % of the average peak value of the previous 10 years at one or more measuring locations in the region or country. The term "airborne allergen-containing particles" means any airborne allergen-containing particle originating from a biological allergen emitting source, including pollen from e.g. grasses, weeds, plants and trees, spores for fungi and any other biological debris from the biological allergen emitting source, which is used by the competent authorities when measuring the level of allergen in the surroundings. The expression "region or country" means the region or country, which is used by the competent authorities when monitoring the level of allergen in the surroundings. The term "peak value" means the average of the three days with the highest values of a season.

The term "SQ-u" means Standardised Quality-Unit: The SQ-u is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

The term "treating" means partly of wholly curing, alleviating symptoms or inhibiting causes of symptoms.

The term "preventing" means any type of prophylactic treatment.

The term "allergy" means any type of hypersensitivity reaction to an environmental allergen mediated by immunological mechanisms, including Type I-IV hypersensitivity reactions, including allergic rhinitis, asthma and atopic dermatitis.

The term "allergen" means any compound capable of eliciting allergy.

In the field of allergy extracts, there is no international accepted standardisation method. A number of different units of extract strength i.e. bio-potency exist. The methods employed and the units used normally measure the allergen content and biological activity. Examples hereof are SQ-Units (Standardised Quality units), BAU (Biological Allergen Units), BU (biological units), UM (Units of Mass), IU (International Units) and IR (Index of Reactivity). Hence, if extracts of origins other than those disclosed herein are used, they need to be standardised against extract disclosed herein in order to determine their potency in SQ units or any of the above mentioned units. The subject matter is dealt with in "Allergenic extracts", H. Ipsen et al, chapter 20 in Allergy, principle and practise (Ed. S. Manning) 1993, Mosby-Year Book, St. Louis and Lowenstein H. (1980) Arb Paul Ehrlich Inst 75:122.

The bio-potency, i.e. the in vivo allergenic activity, of a given extract depends on a number of factors, the most important being the content of major allergens in the extract, which varies with the composition of the biological source material.

The amount of allergen extract in grams to be used for obtaining a desired bio-potency varies with the type of extract in question, and for a given type of extract the amount of allergen extract varies from one batch to another with the actual bio-potency of the extract.

For a given batch of extract, the amount of allergen extract in grams to be used for obtaining a desired bio-potency may be determined using the following procedure:
a) The bio-potency of various amounts of a reference extract is determined using one or more immunological in vivo tests to establish a relationship between bio-potency and amount of reference extract. Examples of the said immunological in vivo tests are Skin Prick Test (SPT), Conjunctival Provocation Test (CPT), Bronchial Challenge with Allergen (BCA) and various clinical trials in which one or more allergy symptoms is monitored, see for example e.g. Haugaard et al., J Allergy Clin Immunol, Vol. 91, No. 3, pp 709-722, March 1993.
b) On the basis of the established relationship between bio-potency and reference extract, the bio-potency of one or more relevant doses for use in the dosage forms of the invention is selected with due consideration to a balance of the factors of i) the effect of treating or alleviating symptoms of allergy, ii) side effects recorded in the immunological in vivo tests, and iii) the variability of i) and ii) from one individual to another. The balancing is done to obtain a maximal adequate therapeutic effect without experiencing an unacceptable level of side effect. The way of balancing the factors are well known to those skilled in the art
   The bio-potency of the one or more relevant doses found may be expressed in any biopotency unit available, such as SQ units, BAU, IR units, IU, cf. above.
c) From the reference extract one or more bio-potency reference standard extracts is prepared and, if used, the bio-potency unit values of the reference standard extracts are calculated on the basis of the bio-potency unit value allocated to the one or more relevant doses, e.g. such a standard for BAU can be obtained from FDA as illustrated below.
d) For the reference standard extracts of each extract type, a number of parameters for evaluating the bio-potency of extracts are selected. Examples of such evaluation parameters are total allergenic activity, the amount of defined major allergens and overall molecular composition of the extract. The total allergenic activity may be measured using an in vitro competitive immunoassay, such as ELISA and MagicLite® luminescence immunoassay (LIA), using a standardised antibody mixture raised against the extract obtained using standard methods, e.g. antibodies raised in mouse or rabbit, or a pool of allergic patients sera. The content of major allergens may e.g. be quantified by rocket immuno-electrophoresis (RIE) and compared to the reference standards. The overall molecular composition may be examined using e.g. crossed immunoelectrophoresis (CIE) and sodium dodecyl sulphate polyacrylamide gel electrophoresis (SDS-PAGE).
e) For a given batch of extract of unknown bio-potency (test extract), the amount of extract to be used for obtaining a desired bio-potency level (effective dose for use in the solid dosage form according to the present invention) may be determined as follows: For each evaluation parameter selected, the test extract is compared with the reference standard extracts using the relevant measurement methods as described above, and on the basis of the measurement results the amount of extract having the desired bio-potency is calculated.

SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity.

BAU (Biological Allergen Units) is biological potency units as determined according to the requirements of the FDA for allergen product described in "Quantitative determination of relative potency of allergenic extracts" ("Methods of the allergen products testing Laboratory" "ELISA competition assay". Page 15, #49N-0012, FDA, October 1993). A dose of 100,000 SQ-Units containing grass extract equals a content of 2600-4700 BAU according to the method above. Likewise, other extracts can be assessed according to the method above.

### EXAMPLES

### EXAMPLE 1: INTRA-SEASONAL UP-DOSING WITH SUBCUTANEOUS ALLERGY VACCINES

### Introduction

In general, in seasonal allergens, such as pollen, a pre-seasonal start of immunotherapy is recommended meaning that immunotherapy has to start e.g. 11-16 weeks prior to the pollen season. This leads to the fact that the patient has to start the treatment when he or she is completely symptom-free and has no feeling that a treatment is necessary. Therefore, many patients do not start immunotherapy because they do not usually go to their physician unless they already have symptoms. An intra-seasonal start of the treatment would allow entering more patients into causal treatment, i.e. immunotherapy, of allergic diseases as opposed to only treating the allergy with symptom-relieving medicaments.

In this Example the safety of an intra-seasonal start of immunotherapy for subcutaneous allergy vaccines has been investigated.

### Study design

This study is based on data relating to intra-seasonal up-dosing in patients with pollen allergy treated in the tree pollen season 2005/06 and grass pollen season 2006. The data include patient data, allergy history of patients, clinical results with up-dosing using vaccines for treating pollen allergy, injection protocols and recorded allergic reactions after the injections. Two types of allergy vaccines was used, 1) an aluminium hydroxide adjuvanted depot vaccine for long term treatment (ALK-depot_SQ) and 2) an aluminium hydroxide adjuvanted depot vaccine for short term treatment (ALK7).

The start of the pollen season was determined according to pollen data from 27 German pollen traps (Stiftung Deutscher Polleninformationsdienst) defining the start of the season as the first of three consecutive days with a pollen count ≥ 10/m³. Data for large local reactions (LLR) and systemic reactions (SR) were analysed by descriptive statistical methods. Data for LLR and SR were additionally compared with patients pre-seasonally treated from another study in a matched pair analysis for the subgroup of patients treated with ALK-depot_SQ for allergy against various grasses and rye. Patients in the age groups ≤15, 16-50, >50 years, with rhinitis or rhinoconjunctivitis without asthma and patients with asthma with or without rhinitis or rhinoconjunctivits were used for comparison. Patients were matched according to age and disease profile. Data were statistically analysed by the Wilcoxon-test and the X²-test.

Data for 134 patients intra-seasonally treated with ALK-depot_SQ pollen products and 12 patients intra-seasonally treated with ALK7 (146 in total) were obtained and evaluated. Some patients were treated with two different allergens making the total number of treatments 152. 23 patients (17.2%) were 15 years or younger. Patients had a mean age of 32.1±16.0 (range 6-76) years, 55.4% were females; in 99.3% of the patients rhinitis was diagnosed, in 87.7% conjunctivitis, in 10.3% asthma and 6.8% atopic dermatitis; 58.2% were treated with grass and rye allergens, 42.5% with tree allergens and 3.4% with mugwort allergens; 90.3% had received no previous specific immunotherapy. A majority of the patients was not severely allergic.

Treatment was started shortly pre-seasonally for 93 treatments (61.2%) and treatment was continued during the season (in the following referred to as pre-seasonally start), the first injection was given intra-seasonally for 59 treatments (38.8%; in the following referred to as intra-seasonally up-dosing). The number of intra-seasonal injections was between 1 and 18 (median 9), median maximum doses were 100 000 SQ-U for ALK-depot SQ and 1 000 SE for ALK7 pollen vaccines. In this Example the terms "pre-seasonally" and "intra-seasonally" mean before and in, respectively, the season of the specific allergen, to which the patients were allergic and with which the patients were treated.

For the treatment with pre-seasonally start, the injections were given weekly both pre-seasonally and intraseasonally. The number of pre-seasonal injections was between 1 and 14 (median 5) and the number of intra-seasonal injections was between 1 and 17 (median 7).

### Results

### Large Local Reactions (LLR)

LLR were recorded only in the group of patients treated with ALK-depot SQ pollen vaccines (8 out of 89 treatments (9.0%) in pre-seasonal injections and 17 out of 140 treatments (12.1 %) during intra-seasonal up-dosing).

**Table 1: Incidence of large local reactions**

| **Injection period** | **No. of LLR per injections** | **% of injections** |
|---|---|---|
| Pre-seasonal start | 14/ 521 | 2.7 |
| Intra-seasonal (up-dosing) | 39/1248 | 3.1 |
| Intra-seasonal (maintenance) | - / 316 | 0.0 |
| Total | 53/2085 | 2.5 |

### Systemic reactions (SR)

SR have been recorded in two patients in total, at intraseasonal up-dosing only one patient had systemic reactions (6 times nausea during intra-seasonal up-dosing).

**Table 2: Incidence of systemic reactions. The 6 SR in intra-seasonal up-dosing were documented in one patient (6 times nausea)**

| **Injection period** | **No. of SR per injections** | **% of injections** |
|---|---|---|
| Pre-seasonal start | 3/ 521 | 0.6 |
| Intra-seasonal (up-dosing) | 6/1248 | 0.5 |
| Intra-seasonal (maintenance) | - / 316 | 0.0 |
| Total | 9/2085 | 0.4 |

### Allergic reactions with need for treatment

Allergic reactions (local and systemic reactions) with need for treatment were only observed in the group of patients treated with ALK-depot SQ pollen products (6 out of 89 patients (6.7%) during pre-seasonal injections and 6 out of 140 patients (4.3%) during intra-seasonal up-dosing).

**Table 3: Frequency of allergic (local and systemic) reactions with need for treatment**

| **Injection period** | **No. of SR per injections** | **% of injections** |
|---|---|---|
| Pre-seasonal start | 12/ 521 | 2.3 |
| Intra-seasonal (up-dosing) | 16/1248 | 1.3 |
| Intra-seasonal (maintenance) | - / 316 | 0.0 |
| Total | 28/2085 | 1.3 |

The medication used for the treatment of LLR and SR was antihistamines and in one case a local corticosteroid in the form of an ointment. No systemic corticosteroids or epinephrine was needed.

### Matched pair comparison for intra-seasonal up-dosing with ALK-depot SQ grass and rye vaccines

The intra-seasonal up-dosing with ALK-depot SQ grass and rye vaccines was compared with matching patients that had been pre-seasonally up-dosed for 80 cases. The comparison within the matched pairs resulted in preferences for intra-seasonal up-dosing with respect to the SR (p=0.0313), but not with respect to local reactions (p=0.0219). The collective comparison based on injections confirmed this tendency.
The frequencies of allergic reactions were:

**Table 4: Comparison of Frequency of LLR in pre-seasonal versus intra-seasonal up-dosing. LLR are more frequent in intra-seasonal up-dosing (p=0.0015).**

| **Injection period** | **No. of LLR per injections** | **% of injections** |
|---|---|---|
| Pre-seasonal up-dosing | 10/641 | 1.6 |
| Intra-seasonal up-dosing | 30/647 | 4.6 |

### Frequency of SR (p=0.0025)

**Table 5: Comparison of Frequency of SR in pre-seasonal versus intra-seasonal up-dosing. SR are more frequent in pre-seasonal up-dosing (p=0.0025).**

| **Injection period** | **No. of SR per injections** | **% of injections** |
|---|---|---|
| Pre-seasonal up-dosing | 9/641 | 1.4 |
| Intra-seasonal up-dosing | -/647 | 0.0 |

### Conclusions

The data of the present study has shown that only in one of 146 patients a mild systemic reaction following intraseasonal up-dosing has been recorded (nausea at 6 injections in one patient). Systemic reactions have been observed in 0.5% of the injections. All reactions (local and systemic) in intra-seasonal up-dosing could be treated with local corticosteroids or local/systemic antihistamines if a treatment was necessary. Systemic corticosteroids or epinephrine was never necessary. No asthmatic reaction following the injections was observed, although 10% of the patients in the study were asthmatics.

A comparison of the patients intra-seasonally up-dosed with ALK-depot SQ grass and rye vaccines with patients that were pre-seasonally up-dosed by matched pair analysis for 80 patients showed a higher frequency of local reactions with intra-seasonal up-dosing (4.6% vs. 1.6%), but no systemic reactions with intra-seasonal up-dosing compared with 9 systemic reactions in the pre-seasonally up-dosed group (0.0% vs. 1.4%).

No previous study is known concerning the safety of an intra-seasonal start of subcutaneous immunotherapy. The present study has shown that an intra-seasonal start of immunotherapy is safe for aluminium hydroxide-based subcutaneous allergy vaccines.

## Claims

1. A vaccine formulation comprising a grass pollen allergen for use in preventing or treating allergy to grass pollen allergen in a subject by subcutaneous administration, wherein the vaccine formulation comprises an adjuvant selected from the group of oxygen-containing metal salts and is administered in a dosage regimen comprising an up-dosing phase, wherein the up-dosing phase is initiated after start of the allergen season of the allergen composition and wherein allergen season means a period of time, the start of which is the first of three consecutive days, wherein the level of airborne allergen-containing particles is above a threshold value, which is 5 % of the average peak value of the previous 10 years, at one or more measuring locations in the region or country, and the end of which is on the last of three consecutive days, wherein the level of airborne allergen-containing particles is below a threshold value, which is 5 % of the average peak value of the previous 10 years at one or more measuring locations in the region or country.

2. The vaccine formulation according to claim 1 for the use according to claim 1, wherein the dosage regimen comprises a maintenance phase following the up-dosing phase.

3. The vaccine formulation according to claim 2, for the use according to claim 2, wherein the duration of the up-dasing phase is from 1 week to 30 weeks.

4. The vaccine formulation according to claim 2 for the use according to claim 2, wherein the duration of the up-dosing phase is 1 week.

5. The vaccine formulation according to any of claims 2-3 for the use according to any of claims 2-3, wherein the administrations in the up-dosing phase are carried out with intervals of from 1 to 14 days.

6. The vaccine formulation according to claim 2, for the use according to claim 2, wherein the administrations in the up-dosing phase are carried out with intervals of 1 day.

7. The vaccine formulation according to claim 1, for the use according to claim 1, wherein the dosage regimen does not comprise a maintenance phase following the up-dosing phase.

8. The vaccine formulation according to claim 7, for the use according to claim 7, wherein the dosage regimen comprises from 1 to 10 up-dosing phases.

9. The vaccine formulation according to claim 7, for the use according to claim 7 wherein the dosage regimen comprises from 3 to 6 up-dosing phases.

10. The vaccine formulation according to claim 2, for the use according to claim 2 wherein the up-dosing phase is initiated more than 1 week after start of the season.

11. The vaccine formulation according to any one of the preceding claims, for the use according to any of the preceding claims wherein the oxygen-containing metal salt is aluminium hydroxide, aluminium phosphate and calcium phosphate.

12. The vaccine formulation according to claim 11, for the use according to claim 11 wherein the oxygen-containing metal salt is aluminium hydroxide.

13. The vaccine formulation according to claim 2, for the use according to claim 2 wherein the up-dosing phase includes a number of dose levels of from 3 to 6.

14. Use of a grass pollen allergen composition for the manufacturing of a vaccine formulation for preventing or treating allergy to grass pollen allergen in a subject by subcutaneous administration, wherein the vaccine formulation comprises an adjuvant selected from the group of oxygen-containing metal salts and is administered in a dosage regimen comprising an up-dosing phase, wherein the up-dosing phase is initiated after start of the allergen season of the allergen composition and wherein allergen season means a period of time, the start of which is the first of three consecutive days, wherein the level of airborne allergen-containing particles is above a threshold value, which is 5 % of the average peak value of the previous 10 years, at one or more measuring locations in the region or country, and the end of which is on the last of three consecutive days, wherein the level of airborne allergen-containing particles is below a threshold value, which is 5 % of the average peak value of the previous 10 years at one or more measuring locations in the region or country.

15. Use according to claim 14, wherein the dosage regimen comprises a maintenance phase following the up-dosing phase.

16. Use according to claim 15, wherein the duration of the up-dosing phase is from 1 week to 30 weeks.

17. Use according to claim 15, wherein the duration of the up-dosing phase is 1 week.

18. Use according to any one of claims 15 to 17, wherein the administrations in the up-dosing phase are carried out with intervals of from 1 to 14 days.

19. Use according to claim 15, wherein the administrations in the up-dosing phase are carried out with intervals of 1 day.

20. Use according to claim 14, wherein the dosage regimen does not comprise a maintenance phase hollowing the up-dosing phase.

21. Use according to claim 20, wherein the dosage regimen comprises from 1 to 10 up-dosing phases.

22. Use according to claim 20, wherein the dosage regimen comprises from 3 to 6 up-dosing phases.

23. Use according to claim 15, wherein the up-dosing phase is initiated more than 1 week after start of the season.

24. Use according to any one of claims 14 to 22, wherein the oxygen-containing metal salt is aluminium hydroxide, aluminium phosphate and calcium phosphate.

25. Use according to claim 24, wherein the oxygen-containing metal salt is aluminium hydroxide.

26. Use according to claim 15, wherein the up-dosing phase includes a number of dose levels of from 3 to 6.

## Patentansprüche

1. Impfstoffformulierung, umfassend ein Graspollenallergen zur Verwendung zur Prävention oder Behandlung einer Allergie gegen Graspollenallergen in einer Person durch subkutane Verabreichung, wobei die Impfstoffformulierung ein Adjuvans umfasst, das ausgewählt ist aus der Gruppe von Sauerstoff enthaltenden Metallsalzen und in einem Dosierungsschema verabreicht wird, welches eine Aufdosierungsphase umfasst, wobei die Aufdosierungsphase nach dem Beginn der Allergensaison der Allergenzusammensetzung begonnen wird und wobei Allergensaison ein Zeitraum bedeutet, dessen Beginn der erste von drei aufeinanderfolgenden Tagen ist, wobei der Level an luftübertragenen allergenhaltigen Partikeln über einem Grenzwert liegt, welcher 5 % des durchschnittlichen Maximalwerts der vorherigen 10 Jahre ist, an einem oder mehreren Messstellen in der Region oder im Land, und dessen Ende an dem letzten von drei aufeinanderfolgenden Tagen ist, wobei der Level an luftübertragenen allergenhaltigen Partikeln unter einem Grenzwert liegt, welcher 5 % des durchschnittlichen Maximalwerts der vorherigen 10 Jahre an einem oder mehreren Messstellen in der Region oder im Land ist.

2. Impfstoffformulierung nach Anspruch 1 zur Verwendung nach Anspruch 1, wobei das Dosierungsschema eine Erhaltungsphase umfasst, welche der Aufdosierungsphase folgt.

3. Impfstofflormulierung nach Anspruch 2 zur Verwendung nach Anspruch 2, wobei die Aufdosierungsphase von 1 Woche bis 30 Wochen dauert.

4. Impfstoffformulierung nach Anspruch 2 zur Verwendung nach Anspruch 2, wobei die Aufdosierungsphase 1 Woche dauert.

5. Impfstoffformulierung nach einem der Ansprüche 2 - 3 zur Verwendung nach einem der Ansprüche 2 - 3, wobei die Verabreichungen in der Aufdosierungsphase mit Intervallen von 1 bis 14 Tagen erfolgen.

6. Impfstoffformulierung nach Anspruch 2 zur Verwendung nach Anspruch 2, wobei die Verabreichungen in der Aufdosierungsphase mit Intervallen von 1 Tag erfolgen.

7. Impfstoffformulierung nach Anspruch 1 zur Verwendung nach Anspruch 1, wobei das Dosierungsschema nicht eine Erhaltungsphase umfasst, welche der Aufdosierungsphase folgt.

8. Impfstoffformulierung nach Anspruch 7 zur Verwendung nach Anspruch 7, wobei das Dosierungsschema 1 bis 10 Aufdosierungsphasen umfasst.

9. Impfstoffformulierung nach Anspruch 7 zur Verwendung nach Anspruch 7, wobei das Dosierungsschema 3 bis 6 Aufdosierungsphasen umfasst.

10. Impfstoffformulierung nach Anspruch 2 zur Verwendung nach Anspruch 2, wobei die Aufdosierungsphase mehr als 1 Woche nach dem Saisonstart begonnen wird.

11. Impfstoffformulierung nach einem der vorangehenden Ansprüche zur Verwendung nach einem der vorangehenden Ansprüche, wobei das Sauerstoff enthaltende Metallsalz Aluminiumhydroxid, Aluminiumphosphat und Kalziumphosphat ist.

12. Impfstoffformulierung nach Anspruch 11 zur Verwendung nach Anspruch 11, wobei das Sauerstoff enthaltende Metallsalz Aluminiumhydroxid ist.

13. Impfstoffformulierung nach Anspruch 2 zur Verwendung nach Anspruch 2, wobei die Aufdosierungsphase eine Anzahl von 3 bis 6 Dosisstufen enthält.

14. Verwendung einer Graspollenallergen-Zusammensetzung zur Herstellung einer Impfstoffformulierung zur Prävention oder Behandlung einer Allergie gegen Graspollenallergen in einer Person durch subkutane Verabreichung, wobei die Impfstoffformulierung ein Adjuvans umfasst, das ausgewählt ist aus der Gruppe von Sauerstoff enthaltenden Metallsalzen und in einem Dosierungsschema verabreicht wird, welches eine Aufdosierungsphase umfasst, wobei die Aufdosierungsphase nach dem Beginn der Allergensaison der Allergenzusammensetzung begonnen wird und wobei Allergensaison ein Zeitraum bedeutet, dessen Beginn der erste von drei aufeinanderfolgenden Tagen ist, wobei der Level an luftübertragenen allergenhaltigen Partikeln über einem Grenzwert liegt, welcher 5 % des durchschnittlichen Maximalwerts der vorherigen 10 Jahre ist, an einem oder mehr Messstellen in der Region oder im Land, und dessen Ende an dem letzten von drei aufeinanderfolgenden Tagen ist, wobei der Level an luftübertragenen allergenhaltigen Partikeln unter einem Grenzwert liegt, welcher 5 % des durchschnittlichen Maximalwerts der vorherigen 10 Jahre an einem oder mehreren Messstellen in der Region oder im Land ist.

15. Verwendung nach Anspruch 14, wobei das Dosierungsschema eine Erhaltungsphase umfasst, welche der Aufdosierungsphase folgt.

16. Verwendung nach Anspruch 15, wobei die Aufdosierungsphase von 1 Woche bis 30 Wochen dauert.

17. Verwendung nach Anspruch 15, wobei die Aufdosierungsphase 1 Woche dauert.

18. Verwendung nach irgendeinem der Ansprüche 15 bis 17, wobei die Verabreichungen in der Aufdosierungsphase mit Intervallen von 1 bis 14 Tagen erfolgen.

19. Verwendung nach Anspruch 15, wobei die Verabreichungen in der Aufdosierungsphase mit Intervallen von 1 Tag erfolgen.

20. Verwendung nach Anspruch 14, wobei das Dosierungsschema nicht eine Erhaltungsphase umfasst, welche der Aufdosierungsphase folgt.

21. Verwendung nach Anspruch 20, wobei das Dosierungsschema 1 bis 10 Aufdosierungsphasen umfasst.

22. Verwendung nach Anspruch 20, wobei das Dosierungsschema 3 bis 6 Aufdosierungsphasen umfasst.

23. Verwendung nach Anspruch 15, wobei die Aufdosierungsphase mehr als 1 Woche nach dem Saisonstart begonnen wird.

24. Verwendung nach irgendeinem der Ansprüche 14 bis 22, wobei das Sauerstoff enthaltende Metallsalz Aluminiumhydroxid, Aluminiumphosphat und Kalziumphosphat ist.

25. Verwendung nach Anspruch 24, wobei das Sauerstoff enthaltende Metallsalz Aluminiumhydroxid ist.

26. Verwendung nach Anspruch 15, wobei die Aufdosierungsphase eine Anzahl von 3 bis 6 Dosisstufen enthält.

## Revendications

1. Une formulation de vaccin comprenant un allergène de pollen de graminée destinée à une utilisation dans la prévention ou le traitement d'une allergie aux allergènes de pollen de graminée chez un sujet par administration sous-cutanée, dans laquelle la formulation de vaccin comprend un adjuvant sélectionné dans le groupe des sels métalliques contenant de l'oxygène et est administrée dans un régime posologique comprenant une phase de progression, dans laquelle la phase de progression est initiée après le début de la saison de l'allergène de la composition d'allergène et dans laquelle la saison de l'allergène signifie un intervalle de temps, dont le début est le premier de trois jours consécutifs, pendant lesquels le niveau des particules contenant des allergènes atmosphériques est au-dessus d'une valeur seuil, qui est de 5% de la valeur de pic moyenne des 10 années précédentes, en un ou plusieurs lieu(x) de mesure de la région ou du pays, et dont la fin est le dernier de trois jours consécutifs, pendant lesquels le niveau des particules contenant des allergènes atmosphériques est au-dessous d'une valeur seuil, qui est de 5% de la valeur de pic moyenne des 10 années précédentes, en un ou plusieurs lieu(x) de mesure de la région ou du pays.

2. La formulation de vaccin selon la revendication 1 pour l'utilisation selon la revendication 1, dans laquelle le régime posologique comprend une phase de maintien suivant la phase de progression.

3. La formulation de vaccin selon la revendication 2 pour l'utilisation selon la revendication 2, dans laquelle la durée de la phase de progression est de 1 semaine à 30 semaines.

4. La formulation de vaccin selon la revendication 2 pour l'utilisation selon la revendication 2, dans laquelle la durée de la phase de progression est d'une semaine.

5. La formulation de vaccin selon la revendication 2 ou 3 pour l'utilisation selon la revendication 2 ou 3, dans laquelle les administrations dans la phase de progression sont effectuées à des intervalles de 1 à 14 jours.

6. La formulation de vaccin selon la revendication 2 pour l'utilisation selon la revendication 2, dans laquelle les administrations dans la phase de progression sont effectuées à des intervalles de 1 jour.

7. La formulation de vaccin selon la revendication 1 pour l'utilisation selon la revendication 1, dans laquelle le régime posologique ne comprend pas de phase de maintien suivant la phase de progression.

8. La formulation de vaccin selon la revendication 7 pour l'utilisation selon la revendication 7, dans laquelle le régime posologique comprend de 1 à 10 phases de progression.

9. La formulation de vaccin selon la revendication 7 pour l'utilisation selon la revendication 7, dans laquelle le régime posologique comprend de 3 à 6 phases de progression.

10. La formulation de vaccin selon la revendication 2 pour l'utilisation selon la revendication 2, dans laquelle la phase de progression est amorcée plus d'une semaine après le début de la saison.

11. La formulation de vaccin selon n'importe laquelle des revendications précédentes pour l'utilisation selon n'importe laquelle des revendications précédentes, dans laquelle le sel métallique contenant de l'oxygène est de l'hydroxyde d'aluminium, du phosphate d'aluminium et du phosphate de calcium.

12. La formulation de vaccin selon la revendication 11 pour l'utilisation selon la revendication 11, dans laquelle le sel métallique contenant de l'oxygène est de l'hydroxyde d'aluminium.

13. La formulation de vaccin selon la revendication 2 pour l'utilisation selon la revendication 2, dans laquelle la phase de progression inclut un nombre de niveaux de dose allant de 3 à 6.

14. Utilisation d'une composition d'allergène de pollen de graminée pour la fabrication d'une formulation de vaccin pour la prévention ou le traitement d'une allergie aux allergènes de pollen de graminée chez un sujet par administration sous-cutanée, dans laquelle la formulation de vaccin comprend un adjuvant sélectionné dans le groupe des sels métalliques contenant de l'oxygène et est administrée dans un régime posologique comprenant une phase de progression, dans laquelle la phase de progression est amorcée après le début de la saison de l'allergène de la composition d'allergène et dans laquelle la saison de l'allergène signifie un intervalle de temps, dont le début est le premier de trois jours consécutifs, pendant lesquels le niveau des particules contenant des allergènes atmosphériques est au-dessus d'une valeur seuil, qui est de 5% de la valeur de pic moyenne des 10 années précédentes, en un ou plusieurs lieu(x) de mesure de la région ou du pays, et dont la fin est le dernier de trois jours consécutifs, pendant lesquels le niveau des particules contenant des allergènes atmosphériques est au-dessous d'une valeur seuil, qui est de 5% de la valeur de pic moyenne des 10 années précédentes, en un ou plusieurs lieu(x) de mesure de la région ou du pays.

15. Utilisation selon la revendication 14, dans laquelle le régime posologique comprend une phase de maintien suivant la phase de progression.

16. Utilisation selon la revendication 15, dans laquelle la durée de la phase de progression est de 1 semaine à 30 semaines.

17. Utilisation selon la revendication 15, dans laquelle la durée de la phase de progression est de 1 semaine.

18. Utilisation selon n'importe laquelle des revendications 15 à 17, dans laquelle les administrations dans la phase de progression sont effectuées à des intervalles de 1 à 14 jours.

19. Utilisation selon la revendication 15, dans laquelle les administrations dans la phase de progression sont effectuées à des intervalles de 1 jour.

20. Utilisation selon la revendication 14, dans laquelle le dosage posologique ne comprend pas de phase de maintien suivant la phase de progression.

21. Utilisation selon la revendication 20, dans laquelle le dosage posologique comprend de 1 à 10 phases de progression.

22. Utilisation selon la revendication 20, dans laquelle le dosage posologique comprend de 3 à 6 phases de progression.

23. Utilisation selon la revendication 15, dans laquelle la phase de progression est amorcée plus d'une semaine après le début de la saison.

24. Utilisation selon n'importe laquelle des revendications 14 à 22, dans laquelle le sel métallique contenant de l'oxygène est de l'hydroxyde d'aluminium, du phosphate d'aluminium et du phosphate de calcium.

25. Utilisation selon la revendication 24, dans laquelle le sel métallique contenant de l'oxygène est de l'hydroxyde d'aluminium.

26. Utilisation selon la revendication 15, dans laquelle la phase de progression inclut un nombre de niveaux de dose allant de 3 à 6.
